# EUROPEAN PATENT SPECIFICATION

(11) **EP 3 092 989 B1**
(45) Date of publication and mention of the grant of the patent: **29.01.2025**
(21) Application number: 14877608.1
(22) Date of filing: 07.07.2014
(51) Int. Cl.: A61L 15/40, A61K 35/28, A61L 27/38, A61L 27/52, A61L 27/60, C12N 5/095

(54) **MESENCHYMAL STEM CELLS-HYDROGEL-BIODEGRADABLE OR MESENCHYMAL STEM CELLS-HYDROGEL-NON-DEGRADABLE SUPPORT COMPOSITION FOR SKIN REGENERATION OR WOUND HEALING**
BIOLOGISCH ABBAUBARE ODER NICHT BIOLOGISCH ABBAUBARE ZUSAMMENSETZUNG AUS MESENCHYMALEN STAMMZELLEN UND HYDROGEL ZUR UNTERSTÜTZUNG DER HAUTREGENERATION ODER WUNDHEILUNG
COMPOSITION DE SUPPORT BIODÉGRADABLE À BASE D'HYDROGEL-CELLULES SOUCHES MÉSENCHYMATEUSES OU NON DÉGRADABLE À BASE D'HYDROGEL-CELLULES SOUCHES MÉSENCHYMATEUSES POUR UNE RÉGÉNÉRATION DE LA PEAU OU UNE CICATRISATION DE PLAIE

(30) Priority: 10.01.2014 KR 20140003499; 30.05.2014 KR 20140065816
(43) Date of publication of application: 16.11.2016
(73) Proprietor: Anterogen Co., Ltd., Geumcheon-gu, Seoul 153-782 (KR)
(72) Inventor: LEE, Sung-Koo, Seoul 153-782 (KR); KIM, Mihyung, Seoul 153-782 (KR); KIM, Inok, Seoul 153-782 (KR)
(74) Representative: Ström & Gulliksson AB
(86) International application number: PCT/KR2014/006068
(87) International publication number: WO 2015/105249

(56) References cited:
- EP-A1- 2 374 485
- WO-A1-2009/155334
- KR-A- 20070 035 592
- KR-A- 20080 032 212
- KR-A- 20090 086 066
- KR-A- 20100 049 341
- KR-A- 20120 126 284
- KR-A- 20130 091 818
- US-A1- 2004 101 518
- US-A1- 2011 053 269
- US-A1- 2011 305 745
- SHANMUGASUNDARAM NATESAN ET AL: "A Bilayer Construct Controls Adipose-Derived Stem Cell Differentiation into Endothelial Cells and Pericytes Without Growth Factor Stimulation", TISSUE ENGINEERING PART A, vol. 17, no. 7-8, 1 April 2011 (2011-04-01), US, pages 941 - 953, XP055385029, ISSN: 1937-3341, DOI: 10.1089/ten.tea.2010.0294
- MAO-SHIH LIANG ET AL: "Engineering fibrin-binding TGF-1 for sustained signaling and contractile function of MSC based vascular constructs", BIOMATERIALS, ELSEVIER SCIENCE PUBLISHERS BV., BARKING, GB, vol. 32, no. 33, 27 July 2011 (2011-07-27), pages 8684 - 8693, XP028295871, ISSN: 0142-9612, [retrieved on 20110801], DOI: 10.1016/J.BIOMATERIALS.2011.07.079

## Description

### [TECHNICAL FIELD]

The present invention relates to a composition comprising mesenchymal stem cell-hydrogel-undegradable support for skin regeneration or wound healing, a sheet comprising the composition and a method for the preparation thereof. Especially, the sheet type composition comprising the stem cells according to the present invention may be used as a dressing for skin regeneration or wound healing.

### [BACKGROUND ART]

Diabetes is a state that glucose level in blood (blood glucose) is higher than that of normal state, which is caused by improper use of ingested food. Diabetic wound, also called as diabetic foot or diabetic foot ulcer, is main complications in diabetic patients and develops from insensibility caused by neuronal cell death due to high blood glucose level and bad blood circulation in diabetic conditions.

Normal wound healing is a series of processes requiring removal of damaged tissues and invaded pathogen followed by reorganization of damaged tissues, which passes through the following processes: (1) inflammatory process in which inflammatory cells such as white blood cells, etc. gather and remove invaded pathogen and dead tissues, etc.; (2) proliferation process in which epithelial cells around the wound migrate and proliferate by the stimulation of growth factors secreted by white blood cells to cover damaged area, and fibrocytes in the dermis accumulate collagen to regenerate capillary blood vessels, resulting in epithelization of the grooved portion; and (3) maturation process in which inflammatory cells disappear and temporarily formed granulation tissues mature into skin tissues similar to original textures. Such restoration processes proceed by the action of various growth factors and cytokines, e.g., Insulin-like Growth Factor (IGF), Transforming Growth Factor beta (TGF-β), Vascular Endothelial Growth Factor (VEGF), basic Fibroblast Growth Factor (bFGF), Platelet-derived Growth Factor (PDGF), Nerve Growth Factor (NGF), Granulocyte-Macrophage Colony Stimulating Factor (GM-CSF), Epidermal Growth Factor (EGF), Hepatocyte Growth Factor (HGF) and through the interaction of the different types of cells such as inflammatory cells, fibroblasts, keratinocytes and endothelial cells, etc.

Normal wound can be easily healed through the successive progression of the above wound healing processes. Skin regeneration or wound healing is conducted through several processes of inflammation, re-epithelialization, granulation, fibroplasia, contraction of wounded tissues, etc. (Freedberg et al., J. Clin. Psychol., 57: 433-455, 2001). Skin regeneration processes are conducted by the cooperation of various cells such as keratinocytes, fibroblasts, endothelial cells, macrophages, platelets, etc, and the complex progresses including migration, infiltration, proliferation and differentiation of such cells are regulated by various growth factors, cytokines and chemokines, and therefore the effectiveness of biological factors or chemicals is well known.

However, the commercialization of the wound healing technique using growth factors and cytokines has some difficulties since the production and isolation of the proteins are very expensive processes. Furthermore, the proteins act complexly in the wound healing processes, so that using only one kind of protein results in a partial alleviation of wound and unsatisfactory responses, which lengthens therapeutic period and makes the treatment ineffective.

In case of chronic wounds of diabetic patients, due to reduced production of growth factors, reduced angiogenesis and degenerated migration and proliferation of keratinocytes and fibroblasts, etc., wound healing processes do not successively proceed but stay in inflammatory process, and regeneration is difficult (Eur J Cell Biol 81:153-60, 2002*;* Br J Surg 90:133-46,2003*).*

In light of the above situation, a skin graft into diabetic wounds using cultured skin and artificial skin composed of fibroblasts and/or epidermal cells composing skin has developed.

Recent study of stem cells revealed that stem cells can be differentiated into skin cells and secrete a large amount of various growth factors since they are highly active compared with skin cells, and also can modulate immune responses. Accordingly, diabetic wound healing technique has been tried by grafting stem cells into wounded area of a diabetic mouse (J Diabetes Res. 2013; 2013:647107, Diabetes. 2013 Jul; 62(7):2588-94, Plast Reconstr Surg. 2011 Oct; 128(4):872-80.). Maharlooei MK et al. (Diabetes Res Clin Pract. 2011 Aug; 93(2):228-34) showed that adult stem cells isolated from adipose tissues have enhanced wound healing activity in diabetic mouse. Furthermore, it has been reported that adipose-derived stem cells were extracted from adipose tissues obtained from abdominal region of diabetic foot ulcer patients by liposuction and then applied to wounded areas without being cultured, which results in complete healing of wounded area of all patients within 8 weeks.

However, the method has a fault that the patients' own tissues should be collected because uncultured cells include immune cells, which is quite inconvenient and even dangerous since wounds occurred in diabetic patients do not heal readily and liposuction may cause another chronic wound. Subculturing of cells isolated from adipose tissues removes immune cells and maintains mesenchymal stem cells which have no immunogenicity, suppress excessive immune response, secrete various growth factors, and can be used in autologous and allogenic transplantation. Such cultured cells have been isolated and applied to diseases, which is called the first generation stem cell therapy products.

Conventional first generation stem cell therapy products are isolated cells obtained by the treatment of protease such as trypsin or dispase. Since protease digests all proteins exposed in cell membrane in a non-selective manner, intercellular connections and basal membrane proteins are rarely maintained after the treatment of protease. Furthermore, mesenchymal stem cells, which are highly adhesive cells, become dead within 6-24 hours after being isolated and have very low cell compatibility.

Korean Patent No. 1,101,321 discloses a hydrogel type cell delivery vehicle for wound healing. The cell delivery vehicle composition is a hydrogel type in which nonionic surfactant polypropylene glycol-polyethylene glycol condensate is dispersed, without gellant, in an aqueous medium selected from the group consisting of saline, phosphate buffer solution (PBS) and cell culture medium in a concentration of 15-50% by weight. The patent document describes the effect of promoting wound healing resulting from moistening and preventing wound contraction, without mentioning the effect of wound healing by the mechanism of promoting the secretion of growth factors or enhancing the level of intercellular substances. Furthermore, it does not disclose the use of biodegradable supports or undegradable supports. Hydrogel has different sizes of network structure, different hardness and decomposition rates depending on manufacturing concentrations, which affect types and proliferation rates of the cells included therein. The patent document does not mention optimal conditions of hydrogel for mesenchymal stem cells.

Considering the above problems, the present inventors have conceived a composition and sheet for skin regeneration or wound healing which comprise highly active living mesenchymal stem cells which are cultured after being dispersed in hydrogel and attached to biodegradable or undegradable supports, and a method for the preparation thereof. Most of the mesenchymal stem cells included in the sheet which is prepared by such method express CD73 and CD90, and at most 70% of the stem cells express CD105.

### [PRIOR ARTS]

Korean Patent No. 1,293,762,
Korean Patent No. 1,106,015,
Korean Patent No. 1,328,604,
Korean Patent No. 1,101,321,
Korean Patent No. 1,335,176
Korean Patent Laid-open Publication No. 2010-0114729,
WO2013/022447,
WO2008/060374,
J Diabetes Res. 2013; 2013:647107,
Diabetes. 2013 Jul; 62(7):2588-94,
Plast. Reconstr. Surg. 2011 Oct; 128(4): 872-80.
Maharlooei MK, et al., Diabetes Res. Clin. Pract. 2011 Aug; 93(2):228-34.;
Tissue eng. (4): 1403-414, 1988

### [PROBLEMS TO BE SOLVED]

The present invention is directed to use of mesenchymal stem cells isolated from human adipose tissues in skin regeneration and wound healing, and the object of the present invention is to provide a composition or a sheet for skin regeneration or wound healing which comprises mesenchymal stem cells from adipose tissue for accomplishing clinically effective therapeutic effects, and a method for the preparation thereof.

### [TECHNICAL MEANS]

Therefore, the present invention provides a composition comprising adipose-derived mesenchymal stem cell-hydrogel-undegradable support, a sheet comprising the composition and a method for the preparation thereof.

The present invention relates to a method of preparing a sheet for skin regeneration or wound healing, comprising the following steps: (a) isolating mesenchymal stem cells from adipose tissue and culturing the stem cells in an expansion medium for at least two passages; (b) combining the cultured adipose-derived mesenchymal stem cells with an undegradable support by using a hydrogel to obtain an adipose-derived mesenchymal stem cell-hydrogel-support; and (c) culturing the adipose-derived mesenchymal stem cell-hydrogel-support obtained in step (b) in an expansion medium. The expansion medium of step (a) or (c) comprises at least one factor selected from the group consisting of FBS (fetal bovine serum), bFGF (basic fibroblast growth factor), EGF (epidermal growth factor), TGF-β1 (transforming growth factor β-1), PDGF (platelet-derived growth factor), VEGF (Vascular endothelial growth factor), HGF (hepatocyte growth factor) and IFG-1(insulin-like growth factor). The hydrogel is one hydrogel selected from the group consisting of fibrin glue, hyaluronic acid, gelatin, alginic acid, cellulose and pectin; and the undegradable support is selected from a sterilized non-woven fabric fiber, PET (polyethylene terephthalate) film, PE (polyethylene) film, PP (polypropylene) film or combinations thereof.

The present invention also relates to a composition for skin regeneration or wound healing, comprising adipose-derived mesenchymal stem cells, a hydrogel and an undegradable support, wherein the hydrogel includes growth factors promoting cell growth and angiogenesis, and an extracellular matrix secreted from the mesenchymal stem cells when the mesenchymal stem cells, hydrogel, and support are cultured in an expansion medium. The hydrogel is one hydrogel selected from the group consisting of fibrin glue, hyaluronic acid, gelatin, alginic acid, cellulose and pectin; and the undegradable support is selected from a sterilized non-woven fabric fiber, PET (polyethylene terephthalate) film, PE (polyethylene) film, PP (polypropylene) film or combinations thereof.

The present invention also relates to a sheet for skin regeneration or wound healing, comprising the composition of the present invention as an active component.

In the composition for skin regeneration or wound healing according to the present invention, the mesenchymal stem cells are autologous or allogenic cells which are positive to CD29, CD44, CD73, CD90 and CD105 while being negative to CD34 and CD45.

According to the present invention, the undegradable support is selected from a sterilized non-woven fabric fiber, PET (polyethylene terephthalate) film, PE (polyethylene) film and PP (polypropylene) film, or a combination thereof.

According to the present invention, the hydrogel is one hydrogel selected from the group consisting of fibrin glue, hyaluronic acid or its derivatives, gelatin, collagen, alginic acid, cellulose and pectin. According to one embodiment, the concentration of fibrinogen composing the fibrin glue is 0.5 to 30 mg/mL, more preferably, 0.5 to 20 mg/mL, still more preferably, 0.5 to 10 mg/mL.

According to an embodiment of the present invention, the method of preparing the sheet for skin regeneration and wound healing comprises the steps of applying a mixture of the stem cells and the hydrogel to the support in an amount of 4,000 to 6,000 cells per cm² of support and culturing the support in an expansion medium comprising FBS and bFGF or EGF until the number of stem cells becomes at least 20,000 cells, more preferably, 20,000 to 200,000 cells per cm² of support.

### [EFFECT OF THE INVENTION]

According to the composition or sheet comprising the mesenchymal stem cells of the present invention, stem cells of high activity may be applied directly to the wound without isolation (selection) process using protease. The composition or sheet has extracellular matrices such as collagen, laminin, fibronectin and elastin secreted by stem cells in the culture stage and included completely in the hydrogel, and therefore it has superior skin regeneration and wound healing effects compared with conventional pharmaceutical preparations and shortens therapeutic period.

More specifically, the adipose-derived mesenchymal stem cell-hydrogel-support according to the present invention maintains fibroblast form in serum-free medium, and at least 90% of the cells survive after one week lapse, which is unexpectedly enhanced survival time compared with conventional stem cell therapy products. When being thawed after cryopreservation, the sheet maintains its initial form and strength and at least 95% of the cells survive, which enables the sheet to be cryopreserved at -80 °C for a long period without cell damage. Since various growth factors and cytokines promoting cell growth and angiogenesis are continuously secreted and lots of various extracellular matrices are secreted and maintained in the hydrogel, the mesenchymal stem cell-hydrogel-support of the present invention provides various extracellular matrices after being transplanted in the body to ease wound healing. Furthermore, the mesenchymal stem cell-hydrogel-support of the present invention does not induce immune response but relieve inflammation by remarkably reducing the secretion of TNF-alpha which is secreted by immunocytes and increases immunoreactivity, and therefore it assists wound healing.

### [BRIEF DESCRIPTION OF DRAWINGS]

FIG. 1a is photographs (x400) of a fluorescence microscope showing the shapes of human adipose-derived mesenchymal stem cells which are cultured with fibrin gel made from fibrinogen stock solution or stepwise dilutions thereof, and then stained with AO/EtBr. The dilution rate of fibrinogen and its concentration in fibrin glue formed from 1:1 mixture with cell suspension containing thrombin are indicated in parentheses.
FIG. 1b is a graph showing the absorbance measured in human adipose-derived mesenchymal stem cells which are cultured with fibrin gel made from fibrinogen stock solution or stepwise dilutions thereof, and then added with WST-1.
FIG. 2a is photographs showing the human adipose-derived mesenchymal stem cell-hydrogel-biodegradable or undegradable support sheet.
FIG. 2b is photographs showing the cells in the sheet of FIG. 2a, in which "a" and "b" are photographs of an optical microscope showing the sheet immediately after the preparation (Day 0) and after being cultured for 5 days, respectively; and "c" is a photograph of a fluorescence microscope showing the sheet after being cultured for 5 days and stained with AO/EtBr.
FIG. 2c is a graph showing the survival rate of cells in the sheet of FIG. 2a over time.
FIG. 3a is a photograph showing the human adipose-derived mesenchymal stem cell-hydrogel-biodegradable support or undegradable support sheet which is thawed after being cryopreserved at -80°C.
FIG. 3b is photographs of a fluorescence microscope showing the cells in the sheet of FIG. 3a after being stained with AO/EtBr, in which "a" shows the mesenchymal stem cells adhered to the biodegradable support or undegradble fiber support; and "b" shows the mesenchymal stem cells adhered to the undegradble film support.
FIG. 4 shows results obtained from a single type cell which is separated from the human adipose-derived mesenchymal stem cell-hydrogel-biodegradable or undegradable support sheet and then measured in flow cytometer after being stained with cell surface proteins. Compared with the result obtained from adipose-derived mesenchymal stem cells subcultured by the method described in prior art, the expressions of CD29, CD44, CD73 and CD90 show similar results while the expression of CD105 decreases to 70% or lower.
FIG. 5a is graphs showing the amounts of VEGF and HGF, quantitatively analyzed by ELISA method, which are secreted by the human adipose-derived mesenchymal stem cell-hydrogel-biodegradable or undegradable support sheet.
FIG. 5b shows results measured in cytokine array kit for angiogenesis stimulating factors secreted by the human adipose-derived mesenchymal stem cell-hydrogel-biodegradable support sheet or undegradable support sheet.
FIG. 6 is photographs showing the human adipose-derived mesenchymal stem cell-hydrogel-biodegradable support sheet or undegradable support sheet which are subjected to fluorescence staining with extracellular matrix proteins.
FIG. 7a is a graph showing the amounts of TNF-α, quantitatively analyzed by ELISA method, which are secreted by peripheral blood mononuclear cells after the co-cultivation of the human adipose-derived mesenchymal stem cell-hydrogel-biodegradable support sheet or undegradable support sheet and allogenic peripheral blood mononuclear cells.
FIG. 7b is a graph showing the suppression rate (%) of secretion of TNF-α, which is obtained from quantitatively analyzing by ELISA method the amount of TNF-α secreted by peripheral blood mononuclear cells after the co-cultivation of the human adipose-derived mesenchymal stem cell-hydrogel-biodegradable support sheet or undegradable support sheet and activated allogenic peripheral blood mononuclear cells, and then converting the amount into the suppression rate.
FIG. 8a is a graph showing the results obtained from diabetic wound models treated with the human adipose-derived mesenchymal stem cell-hydrogel-biodegradable support sheet or undegradable support sheet, vehicle, hydrogel and single type stem cell. In the graph, y-axis indicates a percentage (%) on the initial wound area, and y-axis indicates elapsed days after the treatment.
FIG. 8b is photographs showing the results obtained from diabetic wound models treated with the human adipose-derived mesenchymal stem cell-hydrogel-biodegradable support sheet or undegradable support sheet, vehicle, hydrogel and single type stem cell.

### [BEST MODE TO CARRY OUT THE INVENTION]

In order to achieve the object described above, the present invention provides a composition for skin regeneration or wound healing, which comprises adipose-derived mesenchymal stem cells, a hydrogel and an undegradable support, a sheet comprising the composition and a method for the preparation thereof.

The present invention relates to a method of preparing a sheet for skin regeneration or wound healing, comprising the following steps:
(a) isolating mesenchymal stem cells from adipose tissue and culturing the stem cells in an expansion medium for at least two passages;
(b) combining the cultured adipose-derived mesenchymal stem cells with an undegradable support by using a hydrogel to obtain an adipose-derived mesenchymal stem cell-hydrogel-support; and
(c) culturing the adipose-derived mesenchymal stem cell-hydrogel-support obtained in step (b) in an expansion medium,

wherein the expansion medium of step (a) or (c) comprises at least one factor selected from the group consisting of FBS (fetal bovine serum), bFGF (basic fibroblast growth factor), EGF (epidermal growth factor), TGF-β1 (transforming growth factor β-1), PDGF (platelet-derived growth factor), VEGF (Vascular endothelial growth factor), HGF (hepatocyte growth factor) and IFG-1(insulin-like growth factor);
wherein the hydrogel is one hydrogel selected from the group consisting of fibrin glue, hyaluronic acid, gelatin, alginic acid, cellulose and pectin; and
wherein the undegradable support is selected from a sterilized non-woven fabric fiber, PET (polyethylene terephthalate) film, PE (polyethylene) film, PP (polypropylene) film or combinations thereof.

According to an embodiment of the present invention, the factor included in the expansion medium may be more specifically bFGF (basic Fibroblast Growth Factor), EGF (Epidermal Growth Factor), or combinations thereof.

According to an embodiment of the present invention, the mesenchymal stem cells are autologous or allogenic cells which is positive to CD29, CD44, CD73, CD90, and CD105, while being negative to CD34 and CD45.

According to an embodiment of the present invention, the method of preparing the sheet for wound healing comprises the steps of applying a mixture of the stem cells and the hydrogel to the support in an amount of 4,000 to 6,000 cells per cm² of support and culturing the support in an expansion medium comprising at least one selected from a group consisting of FBS, bFGF and EGF until the number of stem cells becomes at least 20,000 cells, more preferably, 20,000 to 200,000 cells per cm² of support.

According to an embodiment of the present invention, the method can further comprise step (d) activating the cells in step (c) by providing at least one stimulus selected from the group consisting of physical stimulus, hypoxic stimulus, mitogen stimulus and inflammatory factor stimulus, e.g., IFN-v stimulus. In the tissues damaged by diabetic wounds, oxygen supply is not easy due to damaged or deteriorated blood vessels and a chronic inflammation exists, and stem cells applied to the damaged tissues are activated by hypoxic stress and inflammatory factors, so that the secretion of growth factors and cytokines increases sharply. According to the present invention, the composition and sheet comprising highly active stem cells may be prepared by providing a hypoxic stimulus, mitogen or inflammatory factors in the process of manufacturing the support sheet.

According to the present invention, the hydrogel is one hydrogel selected from the group consisting of fibrin glue, hyaluronic acid, gelatin, alginic acid, cellulose or pectin. When fibrin glue is used as the hydrogel, it may comprise fibrinogen in a concentration of 0.5 to 30 mg/mL, preferably, 0.5 to 20 mg/mL, more preferably, 0.5 to 10 mg/mL, still more preferably, 0.5 to 5 mg/mL. The fibrin glue may comprise thrombin in a concentration of 1 to 50 I.U./mL, preferably, 1 to 30 I.U./mL, more preferably, 5 to 20 I.U./mL.

The strength of mesenchymal stem cell-hydrogel sheet is improved by using undegradable support, and the strengthened sheet may be used more conveniently.

According to the present invention, the undegradable support is selected from a sterilized non-woven fabric fiber, PET (polyethylene terephthalate) film, PE (polyethylene) film, PP (polypropylene) film or combinations thereof.

According to an embodiment of the present invention, a combination of at least one biodegradable support and at least one undegradable support may be used. Examples of the combination are PGA (poly-gamma-glutamic acid)/non-woven fabric fiber, PLA (poly lactic acid)/non-woven fabric fiber or PGA/PLA/non-woven fabric fiber.

According to an embodiment of the present invention, the wound can be diabetic wounds.

According to an embodiment of the present invention, the method can further comprise step (e) cryopreserving the adipose-derived mesenchymal stem cell-hydrogel-support in step (c) in a cryopreserving agent comprising 1 to 20 w/v% DMSO and 1 to 50 w/v% human serum albumin, wherein the survival rate of the adipose-derived mesenchymal stem cells is 90% or higher after being thawed.

The present invention also provides a composition for skin regeneration or wound healing, which comprises adipose-derived mesenchymal stem cells, a hydrogel and an undegradable support; wherein the hydrogel includes growth factors promoting cell growth and angiogenesis, and an extracellular matrix secreted from the mesenchymal stem cells when the mesenchymal stem cells, hydrogel, and support are cultured in an expansion medium.

The hydrogel is one hydrogel selected from the group consisting of fibrin glue, hyaluronic acid, gelatin, alginic acid, cellulose and pectin. When fibrin glue is used as the hydrogel, it may comprise fibrinogen in a concentration of 0.5 to 30 mg/mL, preferably, 0.5 to 20 mg/mL, more preferably, 0.5 to 10 mg/mL, still more preferably, 0.5 to 5 mg/mL. The fibrin glue may comprise thrombin in a concentration of 1 to 50 I.U./mL, preferably, 1 to 30 I.U./mL, more preferably, 5 to 20 I.U./mL.

The undegradable support is selected from a sterilized non-woven fabric fiber, PET (polyethylene terephthalate) film, PE (polyethylene) film, PP (polypropylene) film or combinations thereof.

In the composition according to the present invention, a combination of at least one biodegradable support and at least one undegradable support may be used. Examples of the combination are PGA/non-woven fabric fiber, PLA/non-woven fabric fiber or PGA/PLA/non-woven fabric fiber.

In the composition according to the present invention, the wound can be diabetic wounds.

The present invention also provides a sheet for skin regeneration or wound healing, which comprises the above composition as an active component.

### [CONCRETE EXPLANATION TO CARRY OUT THE INVENTION]

According to an embodiment of the present invention, the method comprises more specifically the following steps:
(a) isolating mesenchymal stem cells from adipose tissue and culturing the stem cells in an expansion medium comprising FBS (fetal bovine serum), bFGF (basic fibroblast growth factor) or EGF (epidermal growth factor) for at least two passages;
(b) combining the cultured adipose-derived mesenchymal stem cells with an undegradable support by using a hydrogel to obtain an adipose-derived mesenchymal stem cell-hydrogel-support;
(c) culturing the adipose-derived mesenchymal stem cell-hydrogel-support obtained in step (b) in an expansion medium comprising FBS, bFGF or EGF for about 5 days to prepare a sheet;
(d) activating the cells in step (c) by providing physical stimulus, hypoxic stimulus, mitogen stimulus or inflammatory factor stimulus;
(e) washing the adipose-derived mesenchymal stem cell-hydrogel-support sheet obtained in step (c) or (d) in a medium lacking FBS, bFGF or EGF;
(f) cryopreserving the mesenchymal stem cell-hydrogel-support sheet in step (c) in a cryopreserving agent comprising 10% DMSO and 5% human serum albumin;
(g) thawing the cryopreserved mesenchymal stem cell-hydrogel-support sheet and washing with saline to remove the cryopreserving agent;
(h) applying the sheet cut to a size of wound to a diabetic wound area; and
(i) dressing the sheet.

Hereinafter, the method is explained in more detail.

In step (a), mesenchymal stem cells are isolated from adipose tissue and cultured in an expansion medium for at least two passages according to the method described in Korean Patent No, 1,328,604. By using the expansion medium, a large amount of mesenchymal stem cells may be obtained effectively in a short time. The mesenchymal stem cells obtained through culture for at least two passages according to the prior art adhere to a plastic culture vessel to maintain fibroblast form, and are positive to CD 10, C13, CD29, CD44, CD59, CD71, CD90, CD105 and Oct 4, while being negative to CD34, CD45, CD104, CD106 and Stro-1. The stem cells show multipotency to differentiate into adipocytes, osteocytes, chondrocytes, myocytes, nerve cells, etc. *in vitro.* They also secrete various growth factors such as VEGF, HGF, TGF-β1, NGF and IGF, and have immunomodulatory activity. Accordingly, new techniques for applying stem cells to treat various diseases have been developed.

In step (b), the adipose-derived mesenchymal stem cells obtained through culture for at least two passages in step (a) are treated with trypsin or dispase to obtain single type cells, which are then dispersed in hydrogel. The mixture of stem cells and hydrogel is applied evenly to an undegradable support in an amount of about 5,000 cells/cm² to be adhered thereto. The support is cultured in an expansion medium comprising FBS and bFGF or EGF for about 3 to 5 days. Although fibrin gel is used as a hydrogel in the examples of the present invention, hyaluronic acid, gelatin, alginic acid, cellulose and pectin may be used as well.

Moreover, although a sterilized gauze or PET film is used as an undegradable support, PGA/non-woven fabric fiber or PGA/PLA/non-woven fabric fiber may be used as well.

In the present invention, hydrogel functions primarily to attach mesenchymal stem cells to undegradable supports such as PET film, PE film and PP film. Secondary, it provides substrates for adherent mesenchymal stem cells to make the cells adhered thereto for stable survival. Hydrogel has a lot of three-dimensional network structure (pore), and therefore FBS, bFGF or EGF contained in the medium passes through the pore structure to act on cells. Moreover, hydrogel has different sizes of network structure, hardness and decomposition rates depending on manufacturing concentrations, which affect types and proliferation rates of the cells included therein. In the examples of the present invention, fibrin gel in a final concentration of 0.5 to 10 mg/mL is used to enhance the proliferation rate of the mesenchymal stem cells and to maintain suitable gel strength.

In step (c), the mesenchymal stem cells proliferate rapidly in the mesenchymal stem cell-hydrogel-support sheet to increase fourfold or higher for 3 to 5 days, and therefore the number of stem cells becomes at least 20,000 cells per cm² of support.

According to one embodiment of the present invention, the cells proliferated in the hydrogel express CD29, CD44, CD73, CD90 and CD105, which is characteristic of adipose-derived mesenchymal stem cells, and secrete various growth factors including VEGF and HGF. In addition, the cells have TNF-α and IFN-γ suppressive activities which are representative inflammatory factors secreted in immune cells. That is, the cells cultured in the hydrogel maintain the characteristic of mesenchymal stem cells.

In step (c), hypoxic stress, mitogen treatment or inflammatory factor, e.g. IFN-v treatment may be additionally performed. In the tissues damaged by diabetic wounds, oxygen supply is not easy due to damaged or deteriorated blood vessels and a chronic inflammation exists, and stem cells applied to the damaged tissues are activated by hypoxic stress and inflammatory factors, so that the secretion of growth factors and cytokines increases sharply.

As described above, according to one embodiment of the present invention the method of preparing a sheet comprises highly active stem cells by providing hypoxic stress, mitogen or inflammatory factor in the process of manufacturing the adipose-derived mesenchymal stem cell-hydrogel-support sheet.

The adipose-derived mesenchymal stem cell-hydrogel-support sheet prepared according to the present invention has superior healing ability, because stem cells of high activity may be applied directly to the wound without isolation process using protease. Moreover, the sheet has extracellular matrices such as collagen, laminin, fibronectin and elastin secreted by the mesenchymal stem cells in the culture stage and included completely in the hydrogel, and therefore it may promote wound healing.

The strength of mesenchymal stem cell-hydrogel sheet is improved by using undegradable support, and the strengthened sheet may be used more conveniently. The sheet has a thickness of 0.1-2 mm, which prevents the sheet from tearing when it is applied to wounds, and enables sufficient cells to be included therein for enhancing healing effects.

In step (e), the adipose-derived mesenchymal stem cell-hydrogel-support sheet may be washed with saline two or three times in order to remove animal-derived FBS. The sheet may be further washed with serum-free DMEM medium to remove FBS completely. In this step, FBS is removed from the sheet, which minimizes possible adverse effects resulting from animal-derived components when the sheet is applied to the human body.

The present invention also provides a method of cryopreserving the adipose-derived mesenchymal stem cell-hydrogel-support sheet. The sheet may be preserved at -80°C in Cryo-Bag which is filled with cryopreserving agent consisting of 10% DMSO and 5% human serum albumin solution and then sealed. It is typically known that artificial skins (consisting of epidermal cells, hypodermal cells, or both cells) or isolated cells treated with protease are damaged when being cryopreserved at -80°C, which reduces therapeutic effects when being applied to wounds *(*Tissue eng 4(4): 1403~414, 1988*).* According to the present invention, however, the mesenchymal stem cell-hydrogel-support sheet has stem cells covered by hydrogel, which protects the cells against external impact and stress. Accordingly, the sheet may be preserved at -80 °C without cell damage for a long time.

According to an embodiment of the present invention, the composition of the present invention can be treated with an effective amount of at least one component including growth factors, cytokines, hormones or extracellular matrix compounds or proteins which are useful for enhancing wound healing. Examples of the component are GCSF, IL6, IL8, IL10, MCP1, MCP2, tissue factors, bFGF, KGF, VEGF, PLGF, MMP1, MMP9, TIMP1, TIMP2, TGF-beta1 and HGF, without being limited thereto.

The present invention is explained in more detail by the following Examples. However, these Examples seek to illustrate the present invention only.

### Example 1: Culture method of human adipose-derived mesenchymal stem cells

Adipose tissue is generally obtained by liposuction, without being particularly limited thereto.

Adipose-derived mesenchymal stem cells were isolated from lipoaspirates according to the following procedures: the obtained adipose tissue was washed three or four times using an equal volume of PBS to remove blood therefrom. Adding an equal volume of collagenase solution to the adipose tissue, reaction was carried out in a water bath at 37 °C . The reaction product was placed in a centrifuge tube and centrifuged at 20°C and 1500 rpm for 10 minutes. After removing a fat layer as a supernatant, the remaining collagenase solution as a lower layer was gently separated without being shaken. A stromal medium was added to the collagenase solution to prepare a suspension, followed by centrifugation at 20°C and 1200 rpm for 5 minutes. Here, the settlement part was a stroma-vascular fraction, while the supernatant was discarded. The stroma-vascular fraction was suspended in the stromal medium, inoculated into a culture vessel and cultured for 24 hours in an incubator at 37°C under 5% CO₂. After removing the culture medium and washing with a phosphate buffer, the fraction was proliferated in a stromal medium, a stromal medium containing bFGF in a concentration of 1 ng/mL, or a stromal medium containing EGF in a concentration of 5 ng/mL. When the adipose-derived mesenchymal stem cells were grown to cover 80 to 90% of the culture vessel, the cells were subjected to trypsin treatment to isolate and obtain single type cells.

### Example 2: Determination of the concentration of fibrin glue as a hydrogel

1 mL of Calcium chloride solution was added to freeze dried thrombin to make a concentration of 400∼600 I.U. Alternatively, freeze dried thrombin was thawed and adjusted to the same concentration. Fibrinogen stock solution of a concentration of 71.5-126.5 mg/mL was prepared by adding 1 mL of aprotinin solution to freeze dried fibrinogen, or by thawing freeze dried fibrinogen. The stock solution was stepwise diluted at a ratio of 1:5, 1:10, 1:20 and 1:40. Cells cultured for at least two passages in Example 1 were collected to make a suspension. Thrombin was mixed with the suspension at a ratio of 40~50:1 (v/v), and the mixture was blended with the stepwise diluted fibrinogen at a ratio of 1:1 to form a fibrin gel. After 30 minutes, a culture medium containing 10% FBS and 1 ng/mL bFGF was added to the completely congealed gel, and the mixture was cultured for 5 days in an incubator at 37°C under 5% CO₂. On the second day and fifth day after culturing, cell-fibrin gel mixture was collected and sectioned. The sections were stained with 10 µg/mL acridine orange/ethidium bromide (AO/EtBr), and then the shapes of cells and survival rates were measured by using a fluorescence microscopy. On the fifth day after culturing, WST-1 was added to the culture and cell proliferation condition was determined.

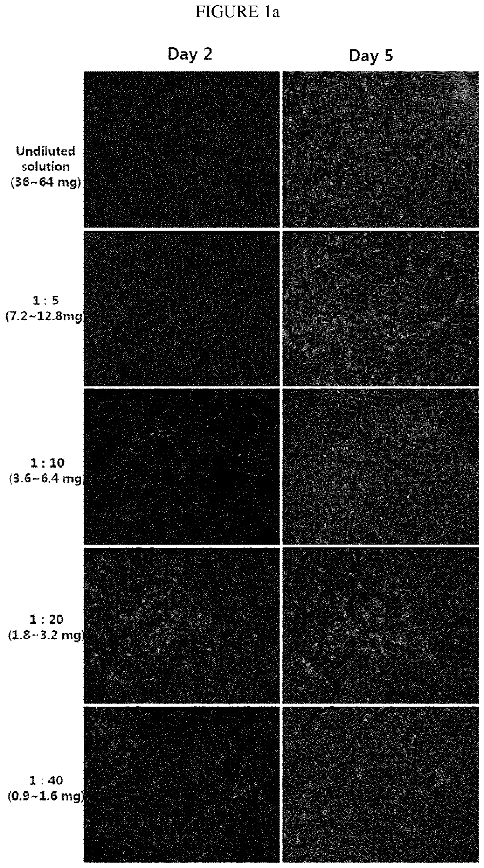

FIG. 1a is photographs of a fluorescence microscope showing the shapes and numbers of stem cells in the fibrin gel prepared from diluted fibrinogen solution. In the fibrin gel made of fibrinogen stock solution, most of cells maintained spherical shape of cells intactly and rarely proliferated. On the other hand, as the dilution rate increased, the cells formed rapidly the shape of fibroblasts and proliferated more considerably. In the fibrin gel (36-64 mg) made of fibrinogen stock solution, some dead cells were observed. In the fibrin gel made of diluted fibrinogen solution, however, no dead cell was found. The results show that fibrin gel of high concentration of 36-64 mg has slight cytotoxicity for adipose-derived mesnchymal stem cells while fibrin gel of the concentration of 18-32 mg or lower has no cytotoxicity.

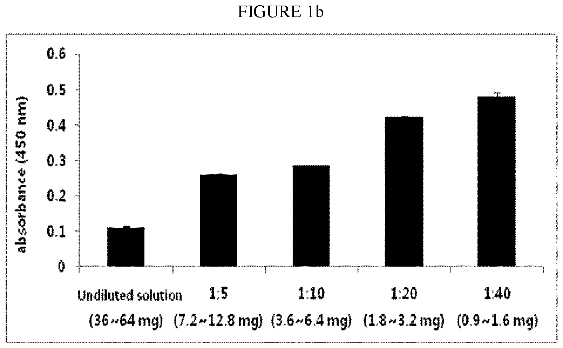

FIG. 1b is a graph showing the proliferation ability of stem cells depending on fibrinogen gel, which is quantitatively determined by using WST-1. As shown in FIG. 1b, the absorbance increases as the dilution rate increases. That is, stem cells proliferate best in the fibrin gel made of fibrinogen diluted by 20 times (3.6-6.3 mg/mL) or 40 times (1.8-3.2 mg/mL).

### Example 3: Preparation of human adipose-derived mesenchymal stem cell-hydrogel-support sheet

Mesenchymal stem cells cultured for at least two passages in Example 1 were collected and added to an expansion medium to make a suspension. Based on the results of Example 2, thrombin was added to the cell suspension to a final concentration of 8∼15 I.U. Fibrinogen of about 3-6.5 mg/mL was uniformly coated on biodegradable support, vicryl mesh or bovine amniotic membrane, or undegradable support, gauze or PET film of 10x10 cm² sized square. Afterward, the cell suspension containing thrombin was coated on the support in an amount of about 5,000 cells/cm², and the resultant was gently shaken up and down for the cell-fibrin gel to be uniformly formed and adhered to the support. After 30 minutes, an expansion medium was added to the completely congealed gel, and the mixture was cultured for 3-5 days in an incubator at 37°C under 5% CO₂.

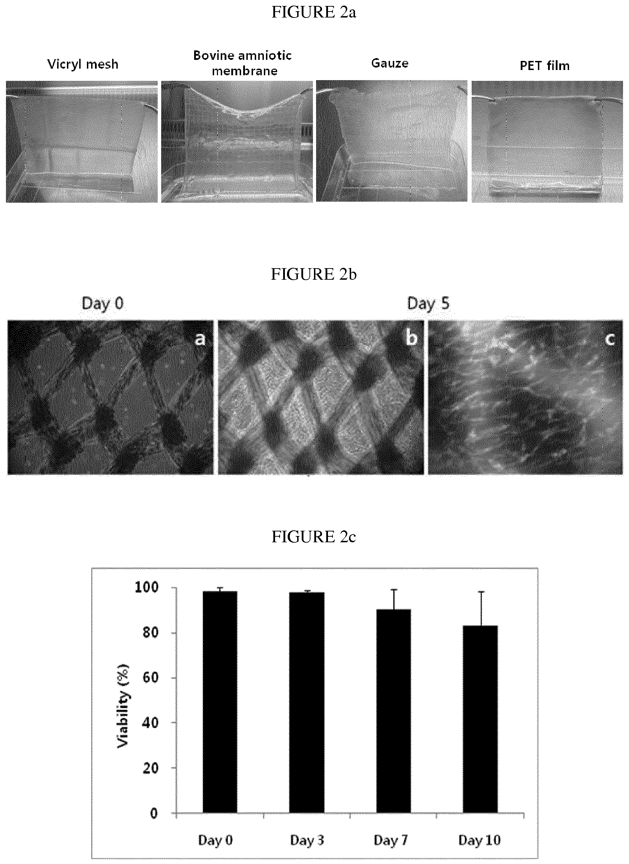

FIG. 2a is photographs showing the sheet prepared by mixing human adipose-derived mesenchymal stem cells with fibrin hydrogel, attaching the mixture to a biodegradable support, vicryl mesh or bovine amniotic membrane, or undegradable support, gauze or PET film sheet, and then culturing the resultant. According to the present invention, the strength of sheet is improved by attaching the cell-hydrogel to biodegradable or undegradable support to make a sheet, and the strengthened sheet may be used more conveniently. The cell-hydrogel-biodegradble or undegradable sheet has a thickness of 0.1-2 mm, which prevents the sheet from tearing when it is clinically applied to wounds, and enables sufficient cells to be included therein for enhancing healing effects.

FIG. 2b is photographs of an optical microscope or a fluorescence microscope showing the sheet after being stained with AO/EtBr. As shown in FIG. 2b, spherical adipose-derived mesenchymal stem cells were distributed at a low concentration in the support on day 0. On the other hand, after culturing for 5 days, fibroblast-type cells were observed to be adhered to the support and proliferate. About 20,000-60,000 cells were uniformly distributed per cm² of sheet and 100% of the cells survived.

According to another embodiment, the sheet cultured for 5 days were washed with saline to remove FBS, and serum-free DMEM was added thereto, and then the mixture was left at 37 °C for 10 days. On the third day, fifth day, seventh day and tenth day, survival rates of the cells were determined after staining with EtBr/AO. As a result, adipose-derived mesenchymal stem cells in the sheet prepared according to the present invention maintained fibroblast-type shape even in a serum-free medium, and at least 98% survived on the third day, at least 90% survived on the seventh day, and at least 80% survived on the tenth day, as shown in FIG. 2c.

### Example 4: Cryopreservation of human adipose-derived mesenchymal stem cell-hydrogel-support sheet

The human adipose-derived mesenchymal stem cell-hydrogel-biodegradable or undegradable support sheet prepared in Example 3 was washed to remove cell culture medium, and then cryopreserved at -80 °C in Cryo-Bag filled with cryopreserving agent (10% DMSO and human serum albumin solution). After about 1 month, the Cryo-Bag was immersed in a constant-temperature water bath of 37 °C, and then the melted cryopreserving agent was drained. Saline was added thereto, shaken up and down, and then drained. After the remaining cryopreserving agent was completely removed by additional 1~3 times washing, the sheet was stained with AO/EtBr and then survival rate was determined.

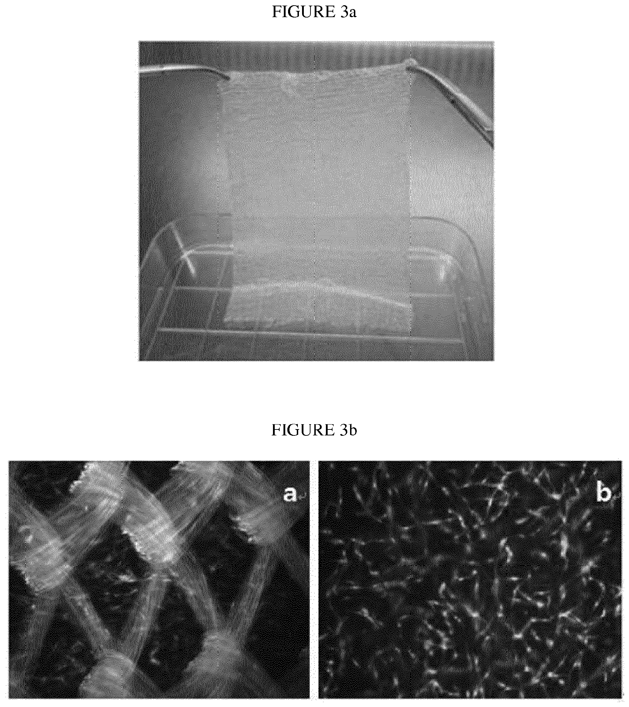

As shown in FIG. 3, the human adipose-derived mesenchymal stem cell-hydrogel-biodegradable or undegradable support sheet which is thawed after being cryopreserved at -80°C maintained the initial shape and strength of the sheet. Furthermore, at least 95% cells in the sheet survived. The results show that the human adipose-derived mesenchymal stem cell-hydrogel-biodegradable or undegradable support sheet prepared according to the present invention can be cryopreserved at -80°C without cell damage for a long time.

### Experimental Example 1: Biological characteristics of human adipose-derived mesenchymal stem cell-hydrogel-support

The adipose-derived mesenchymal stem cells isolated and cultured in Example 1 according to prior art (Korean Patent No. 1,328,604) are positive to CD10, C13, CD29, CD44, CD59, CD71, CD90, CD105 and Oct 4, and negative to CD34, CD45, CD104, CD106 and Stro-1. The sheet prepared in Example 3 by using the cells obtained in Example 1 was treated by enzyme to dissolve fibrin glue and to isolate single type cells. The collected cells were placed in a 1.5 mL-centrifuge tube. After adding 1 mL of a FACS staining solution (phosphate buffer solution containing 1% fetal bovine serum) thereto and homogeneously mixing the same, centrifugation at 10,000 rpm was conducted for 5 seconds. After removing the supernatant, the remaining product was suspended in 1mL of FACS staining solution and centrifuged at 10,000 rpm for 5 seconds. After removing the supernatant, the remaining product was re-suspended in 300*µ*ℓ of the FACS staining solution. The resultant sample was distributed into new centrifuge tubes, such that about 0.5 to 1.0×10⁶ cells were placed in each of the centrifuge tubes, depending on the number of samples. After adding an antibody thereto, the content in the tube was subjected to reaction in ice bath for 30 minutes. Then, the reaction product was re-suspended in 1mL of the FACS staining solution and centrifuged at 10,000 rpm for 5 seconds, followed by removal of the supernatant. The remaining product was re-suspended by adding 400 to 500 *µ*ℓ of a FACS fixing solution thereto. The obtained suspension was subjected to analysis using a flow cytometer.

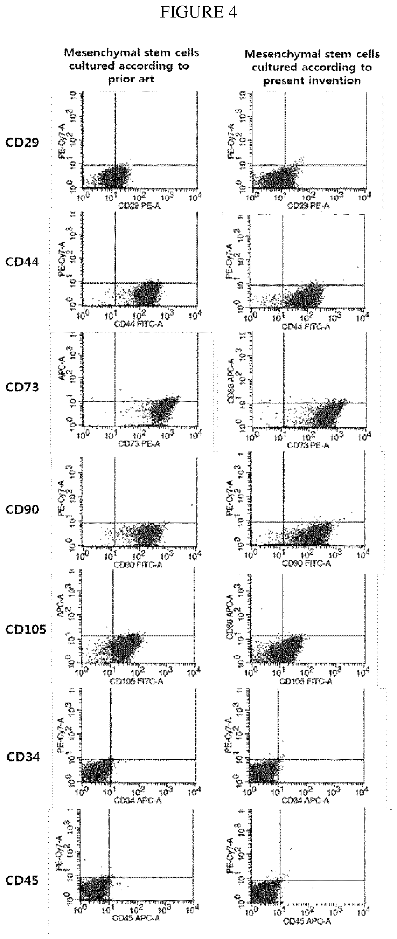

As a result, the adipose-derived mesenchymal stem cells cultured in hydrogel according to the present invention still maintain the immunological characteristics which show positive response to CD29, CD44, CD73, CD90 and CD105, and negative response to CD34 and CD45, as shown in FIG. 4. The expression of CD105 decreases to 70% or lower, which is reduction by 20% or higher compared with the result obtained from the stem cells cultured by the method described in the prior art.

### Experimental Example 2: Secretion of growth factors by human adipose-derived mesenchymal stem cell-hydrogel-support

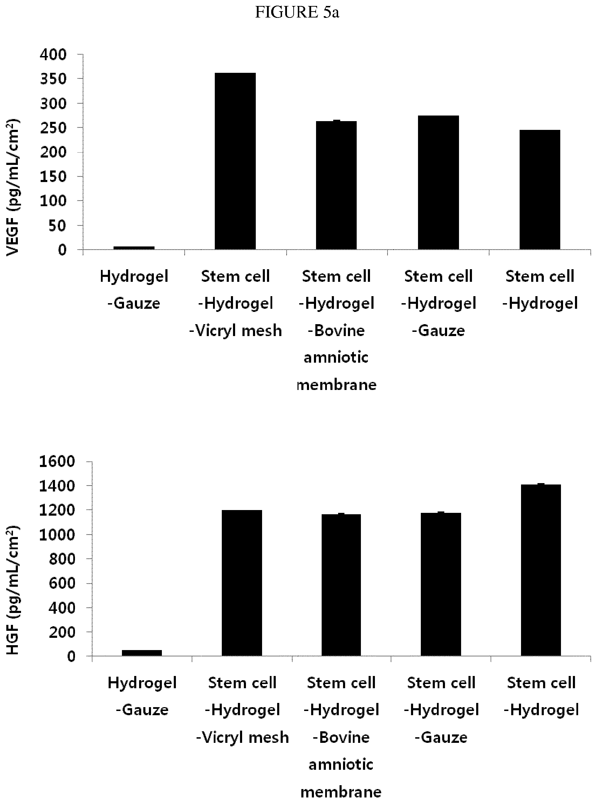

The human adipose-derived mesenchymal stem cell-hydrogel-biodegradable or undegradable support sheet prepared in Example 3 or the cryopreserved sheet of Example 4 after being thawed was washed with PBS and cut to a size of 0.8x0.8 cm³. Two sheets were placed in a 24-well plate, and 1 mL of DMEM was added thereto. After culturing for 72 hours in an incubator at 37 °C under 5% CO₂, supernatant was collected and then determined the amounts of VEGF and HGF, which are representative growth factors secreted by mesenchymal stem cells, by using ELISA method. As a result, it is confirmed that the sheets secrete HGF and VEGF, as shown in FIG. 5a.

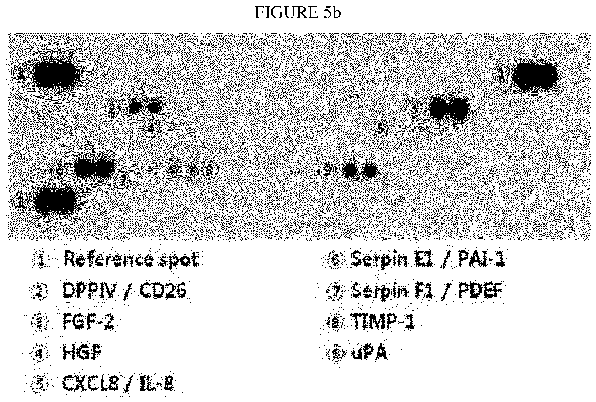

According to another embodiment, the collected supernatant was analyzed by using angiogenesis-related cytokine array kit. As a result, it is confirmed that the sheets secrete growth factors including HGF and VEGF and various cytokines such as Serpin E1 (PAI-1) and F1 (PDEF), TIMP-1, CXCL8 (IL-8), FGF-2 and DPPIV (CD26), which promote angiogenesis, as shown in FIG 5b. The results show that the mesenchymal stem cells cultured by using hydrogel and biodegradable or undegradable support according to the present invention facilitate wound healing by secreting continuously various growth factors and cytokines which promote cell proliferation and angiogenesis.

### Experimental Example 3: ECM secretion activity of human adipose-derived mesenchymal stem cell-hydrogel-support

The human adipose-derived mesenchymal stem cell-hydrogel-biodegradable or undegradable support sheet prepared in Example 3 was made into frozen sections and then fixed by using PBS containing 3.7% formaldehyde for 30 minutes. After washing the fixed sections three times with PBS, the washed product was subjected to permeabilization and blocking by using PBS containing 5% normal goat serum and 0.1% triton X-100 for 30 minutes. After adding PBS containing a primary antibody thereto and reacting at 37°C for 1 hour, the reaction product was washed three times with PBS and reacted with a secondary antibody at room temperature for 30 minutes. After the product was washed again three times with PBS, the washed product was mounted and observed using a fluorescence microscope.

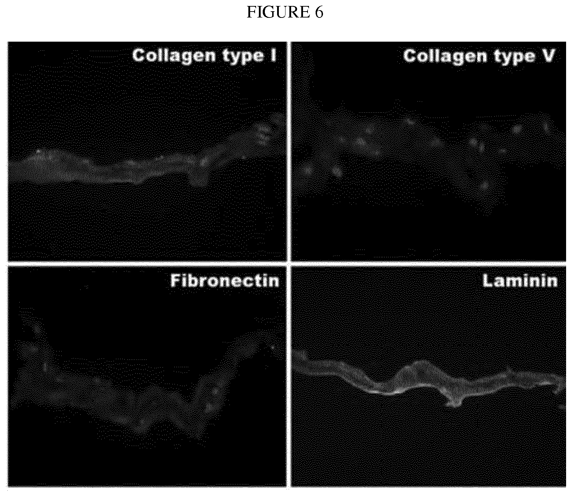

FIG. 6 is photographs (×400) showing extracellular matrix (ECM) protein secretion activity of the adipose-derived mesenchymal stem cells in the sheet. As shown in the photographs, the adipose-derived mesenchymal stem cell-hydrogel-biodegradable or undegradable support according to the present invention generally exhibited positive response to collagen types I, fibronectin and laminin. That is, the cells composing the adipose-derived mesenchymal stem cell-hydrogel-biodegradable or undegradable support prepared according to the present invention may secrete lots of various types of extracellular matrices, which remain in hydrogel and facilitate wound healing when being transplanted into a body.

### Experimental Example 4: Immunomodulatory activity of allogenic human adipose-derived mesenchymal stem cell-hydrogel-support

The human adipose-derived mesenchymal stem cell-hydrogel-biodegradable or undegradable support sheet prepared in Example 3 or the cryopreserved sheet of Example 4 after being thawed was washed with PBS and cut to a size of 0.8x0.8 cm³. Each sheet was placed in a 24-well plate. Peripheral blood mononuclear cells (PBMC) obtained from a donor of different human leukocyte antigen (HLA) were added to the 24-well plate to be 5×10⁵ cells/well. As a positive control, peripheral blood mononuclear cells were treated with mitogen phyto-hemagglutinin (PHA) to induce immune response of peripheral blood mononuclear cells. On the third day after reaction, a supernatant was collected and subjected to measurement of an amount of secreted TNF-α by using ELISA method.

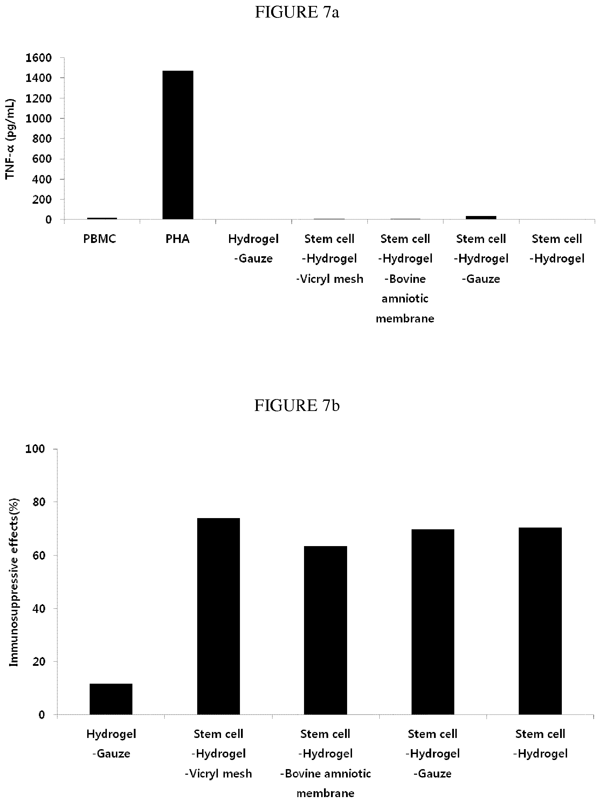

FIG. 7a is a graph showing the immunogenicity of the allogenic human adipose-derived mesenchymal stem cell-hydrogel-biodegradable or undegradable support. As shown in the graph, the positive control, peripheral blood mononuclear cells were activated by PHA, while in the reaction with the allogenic human adipose-derived mesenchymal stem cell-hydrogel-biodegradable or undegradable support, TNF-α was rarely secreted. That is, it can be seen that the allogenic human adipose-derived mesenchymal stem cell-hydrogel-biodegradable or undegradable support does not induce immune response.

According to another embodiment, 5×10⁵ peripheral blood mononuclear cells were added to a 24-well plate, and activated with PHA to induce immune response. The allogenic human adipose-derived mesenchymal stem cell-hydrogel-biodegradable or undegradable support sheet prepared in Example 3 or 4 was cut to a size of 0.8x0.8 cm³ and each sheet was placed in the plate. On the third day after reaction, a supernatant was collected and subjected to measurement of an amount of secreted TNF-α.

As a result, the allogenic human adipose-derived mesenchymal stem cell-hydrogel-biodegradable or undegradable support sheet in peripheral blood mononuclear cells activated by PHA reduced the amount of secreted TNF-α by at least 60%, as shown in FIG. 7b. That is, it can be seen that the allogenic human adipose-derived mesenchymal stem cell-hydrogel-biodegradable or undegradable support sheet does not induce immune response, and considerably decreases secretion of TNF-α which is secreted in large quantities by immune cells when immune response occurs to thus increase immune activity. Accordingly, when applied to diabetic wounds, the adipose-derived mesenchymal stem cell-hydrogel-biodegradable or undegradable support sheet can relieve chronic inflammation of wound and promote wound healing.

### Experimental Example 5: Wound healing effect of adipose-derived mesenchymal stem cell-hydrogel-support sheet

Male db/db diabetic mice obtained from SLC (Japan) were acclimated until the blood glucose becomes 250 mg/dL or higher. For surgery, a 1:1 mixture of Rompun and Zoletil 50 was administered intraperitoneally to the mice. After removing hairs on the dorsal zone of the anesthetized mice with a depilator, the zone was disinfected with isopropanol. A circular biopsy wound having a diameter of 8 mm was made on the both sides of the dorsal zone in each mouse, and a silicone splint was attached thereto for maintaining the wound and preventing contraction of the wound. The wound was taken a photograph by a digital camera. The human adipose-derived mesenchymal stem cell-hydrogel-biodegradable or undegradable support sheet prepared in Example 3 was cut to the same size as the wound and covered on the wound. The wound was dressed by Tegaderm to be covered fully, and then dressed by an elastic support bandage thereon. On the fifth day, after carefully removing Tegaderm, the wound was taken a photograph and then covered by Tegaderm again. Afterward, the wound was taken a sequential photographs every three days for about two weeks. The wound area was calculated by using NIH image processing program ImageJ, and then converted into a percentage (%) based on initial wound area.

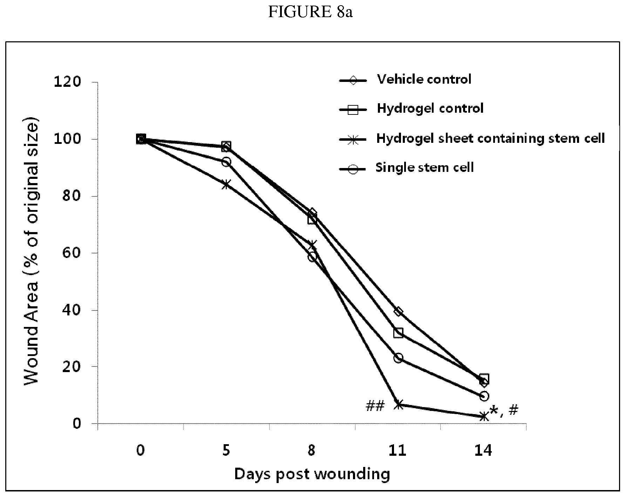

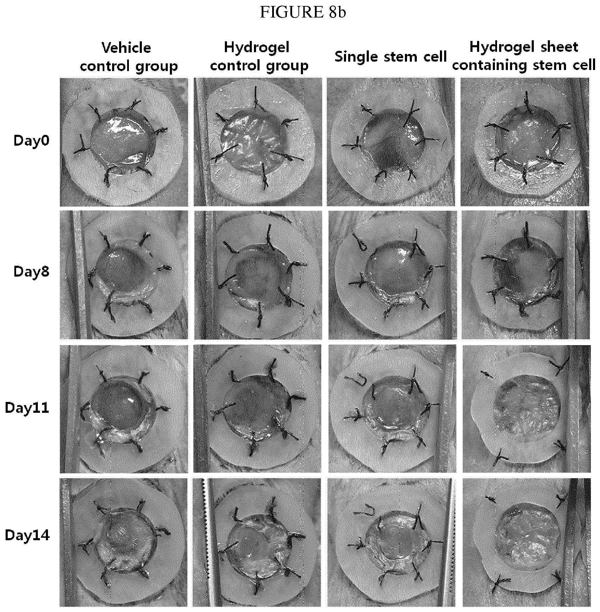

The result showed a tendency that the wound size of the group treated with the human adipose-derived mesenchymal stem cell-hydrogel-biodegradable or undegradable support sheet reduced compared with that of the vehicle control group or hydrogel control group by the eighth day after the wound formation, but showing no significant differences, as shown in FIG. 8a. From the eleventh day, however, the wound size of the group treated with the human adipose-derived mesenchymal stem cell-hydrogel-biodegradable or undegradable support sheet reduced significantly compared with that of the vehicle control group or hydrogel control group. On the fourteenth day, the wounds of most of the groups treated with the human adipose-derived mesenchymal stem cell-hydrogel-biodegradable or undegradable support sheet were completely closed, while those of the vehicle control group, hydrogel control group and single cell-treated group remained in most cases except some cases. The wound size of the group treated with a single type cell cultured by prior art method reduced compared with that of the control group, but showing no significant differences. That is, it can be seen that the single type human adipose-derived mesenchymal stem cells cultured by prior art method have incomplete healing effect on diabetic wound, while the human adipose-derived mesenchymal stem cell-hydrogel-biodegradable or undegradable support sheet has superior healing effect on diabetic wound.

### [INDUSTRIAL APPLICBILITY]

The composition or sheet comprising highly active mesenchymal stem cells according to the present invention has superior skin regeneration and wound healing effects and shortens therapeutic period compared with conventional pharmaceutical preparations, since stem cells of high activity may be applied directly to the wound without isolation (selection) process using protease, and the extracellular matrices such as collagen, laminin, fibronectin and elastin secreted by stem cells in the culture stage are included completely in the hydrogel, and therefore it can be effectively used as a composition or sheet for skin regeneration and wound healing.

## Claims

1. A method of preparing a sheet for skin regeneration or wound healing, comprising the following steps:
(a) isolating mesenchymal stem cells from adipose tissue and culturing the stem cells in an expansion medium for at least two passages;
(b) combining the cultured adipose-derived mesenchymal stem cells with an undegradable support by using a hydrogel to obtain an adipose-derived mesenchymal stem cell-hydrogel-support; and
(c) culturing the adipose-derived mesenchymal stem cell-hydrogel-support obtained in step (b) in an expansion medium,
wherein the expansion medium of step (a) or (c) comprises at least one factor selected from the group consisting of FBS (fetal bovine serum), bFGF (basic fibroblast growth factor), EGF (epidermal growth factor), TGF-β1 (transforming growth factor β-1), PDGF (platelet-derived growth factor), VEGF (Vascular endothelial growth factor), HGF (hepatocyte growth factor) and IFG-1(insulin-like growth factor);
wherein the hydrogel is one hydrogel selected from the group consisting of fibrin glue, hyaluronic acid, gelatin, alginic acid, cellulose and pectin; and
wherein the undegradable support is selected from a sterilized non-woven fabric fiber, PET (polyethylene terephthalate) film, PE (polyethylene) film, PP (polypropylene) film or combinations thereof.

2. The method according to claim 1, wherein the fibrin glue comprises fibrinogen in a concentration of 0.5 to 30 mg/mL.

3. The method according to claim 1, wherein the fibrin glue comprises fibrinogen in a concentration of 0.5 to 10 mg/mL.

4. The method according to claim 1, wherein the fibrin glue comprises thrombin in a concentration of 1 to 50 I.U./mL.

5. The method according to claim 1, further comprising step (d) activating the cells in step (c) by providing at least one stimulus selected from the group consisting of physical stimulus, hypoxic stimulus, mitogen stimulus and inflammatory factor stimulus.

6. The method according to claim 1, the wound is a diabetic wound.

7. The method according to claim 1, further comprising step (e) cryopreserving the adipose-derived mesenchymal stem cell-hydrogel-support in step (c) in a cryopreserving agent comprising 1 to 20 w/v% DMSO (dimethyl sulfoxide) and 1 to 50 w/v% human serum albumin, wherein the survival rate of the adipose-derived mesenchymal stem cells is 90% or higher after being thawed.

8. A composition for skin regeneration or wound healing, comprising adipose-derived mesenchymal stem cells, a hydrogel and an undegradable support,
wherein the hydrogel includes growth factors promoting cell growth and angiogenesis, and an extracellular matrix secreted from the mesenchymal stem cells when the mesenchymal stem cells, hydrogel, and support are cultured in an expansion medium;
wherein the hydrogel is one hydrogel selected from the group consisting of fibrin glue, hyaluronic acid, gelatin, alginic acid, cellulose and pectin; and
wherein the undegradable support is selected from a sterilized non-woven fabric fiber, PET (polyethylene terephthalate) film, PE (polyethylene) film, PP (polypropylene) film or combinations thereof.

9. The composition according to claim 8, wherein the fibrin glue comprises fibrinogen in a concentration of 0.5 to 30 mg/mL.

10. The composition according to claim 8, wherein the fibrin glue comprises fibrinogen in a concentration of 0.5 to 10 mg/mL.

11. The composition according to claim 8, wherein the fibrin glue comprises thrombin in a concentration of 1 to 50 I.U./mL.

12. The composition according to claim 8, the wound is a diabetic wound.

13. A sheet for skin regeneration or wound healing, comprising one of the composition of claims 8 to 12 as an active component.

## Patentansprüche

1. Verfahren zur Herstellung einer Folie für Hautregeneration oder Wundheilung, umfassend die folgenden Schritte:
(a) Isolieren mesenchymaler Stammzellen aus Fettgewebe und Kultivieren der Stammzellen in einem Expansionsmedium für wenigstens zwei Passagen;
(b) Kombinieren der kultivierten, aus Fett gewonnenen mesenchymalen Stammzellen mit einem nicht-abbaubaren Träger durch Verwendung eines Hydrogels, um einen Träger aus aus Fett gewonnenen mesenchymalen Stammzellen und Hydrogel zu erhalten; und
(c) Kultivieren des in Schritt (b) erhaltenen Trägers aus aus Fett gewonnenen mesenchymalen Stammzellen und Hydrogel in einem Expansionsmedium, wobei das Expansionsmedium von Schritt (a) oder (c) wenigstens einen Faktor umfasst, der ausgewählt ist aus der Gruppe, bestehend aus FBS (fötalem Rinderserum), bFGF (basischem Fibroblasten-Wachstumsfaktor), EGF (epidermalem Wachstumsfaktor), TGF-β1 (transformierendem Wachstumsfaktor β-1), PDGF (aus Blutplättchen gewonnenem Wachstumsfaktor), VEGF (vaskulärem endothelialem Wachstumsfaktor), HGF (Hepatozyten-Wachstumsfaktor) und IFG-1 (insulinähnlichem Wachstumsfaktor);
wobei das Hydrogel ein Hydrogel ist, das ausgewählt ist aus der Gruppe, bestehend aus Fibrinkleber, Hyaluronsäure, Gelatine, Alginsäure, Zellulose und Pektin; und
wobei der nicht-abbaubare Träger ausgewählt ist aus einem sterilisierten Vliesfaserstoff, PET(Polyethylenterephthalat)-Film, PE(Polyethylen)-Film, PP(Polypropylen)-Film oder Kombinationen davon.

2. Verfahren nach Anspruch 1, wobei der Fibrinkleber Fibrinogen in einer Konzentration von 0,5 bis 30 mg/mL umfasst.

3. Verfahren nach Anspruch 1, wobei der Fibrinkleber Fibrinogen in einer Konzentration von 0,5 bis 10 mg/mL umfasst.

4. Verfahren nach Anspruch 1, wobei der Fibrinkleber Thrombin in einer Konzentration von 1 bis 50 I.E./mL umfasst.

5. Verfahren nach Anspruch 1, das weiter Schritt (d) umfasst, in dem die Zellen in Schritt (c) durch Bereitstellen wenigstens eines Stimulus aktiviert werden, der ausgewählt ist aus der Gruppe, bestehend aus physischem Stimulus, hypoxischem Stimulus, mitogenem Stimulus und Stimulus durch Entzündungsfaktor.

6. Verfahren nach Anspruch 1, wobei die Wunde eine diabetische Wunde ist.

7. Verfahren nach Anspruch 1, das weiter Schritt (e) umfasst, in dem der Träger aus aus Fett gewonnenen mesenchymalen Stammzellen und Hydrogel in Schritt (c) in einem Kältekonservierungsmittel, das 1 bis 20 w/v% DMSO (Dimethylsulfoxid) und 1 bis 50 w/v% Humanserumalbumin umfasst, kältekonserviert wird, wobei die Überlebensrate der aus Fett gewonnenen mesenchymalen Stammzellen 90% oder höher ist, nachdem sie aufgetaut sind.

8. Zusammensetzung zur Hautregeneration oder Wundheilung, umfassend aus Fett gewonnene mesenchymale Stammzellen, ein Hydrogel und einen nicht-abbaubaren Träger,
wobei das Hydrogel Wachstumsfaktoren einschließt, die Zellwachstum und Angiogenese fördern, und eine extrazelluläre Matrix, die aus den mesenchymalen Stammzellen sekretiert wird, wenn die mesenchymalen Stammzellen, Hydrogel und Träger in einem Expansionsmedium kultiviert werden;
wobei das Hydrogel ein Hydrogel ist, das ausgewählt ist aus der Gruppe, bestehend aus Fibrinkleber, Hyaluronsäure, Gelatine, Alginsäure, Zellulose und Pektin; und
wobei der nicht-abbaubare Träger ausgewählt ist aus einem sterilisierten Vliesfasergewebe, PET(Polyethylenterephthalat)-Film, PE(Polyethylen)-Film, PP(Polypropylen)-Film oder Kombinationen davon.

9. Zusammensetzung nach Anspruch 8, wobei der Fibrinkleber Fibrinogen in einer Konzentration von 0,5 bis 30 mg/mL umfasst.

10. Zusammensetzung nach Anspruch 8, wobei der Fibrinkleber Fibrinogen in einer Konzentration von 0,5 bis 10 mg/mL umfasst.

11. Zusammensetzung nach Anspruch 8, wobei der Fibrinkleber Thrombin in einer Konzentration von 1 bis 50 I.E./mL umfasst.

12. Zusammensetzung nach Anspruch 8, wobei die Wunde eine diabetische Wunde ist.

13. Folie für Hautgeneration oder Wundheilung, umfassend eine der Zusammensetzungen nach Anspruch 8 bis 12 als eine aktive Komponente.

## Revendications

1. Procédé de préparation d'une feuille pour la régénération de la peau ou la cicatrisation de plaies, comprenant les étapes suivantes :
(a) l'isolement de cellules souches mésenchymateuses issues de tissu adipeux et la mise en culture des cellules souches dans un milieu d'expansion pendant au moins deux passages ;
(b) la combinaison des cellules souches mésenchymateuses dérivées de tissu adipeux mises en culture avec un support non dégradable en utilisant un hydrogel pour obtenir un support cellules souches mésenchymateuses dérivées de tissu adipeux-hydrogel ; et
(c) la mise en culture du support cellules souches mésenchymateuses dérivées de tissu adipeux-hydrogel obtenu à l'étape (b) dans un milieu d'expansion,
dans lequel le milieu d'expansion de l'étape (a) ou (c) comprend au moins un facteur choisi dans le groupe consistant en FBS (sérum foetal bovin), bFGF (facteur de croissance basique des fibroblastes), EGF (facteur de croissance épidermique), TGF-β1 (facteur de croissance transformant β-1), PDGF (facteur de croissance dérivé des plaquettes), VEGF (facteur de croissance endothéliale vasculaire), HGF (facteur de croissance des hépatocytes) et IFG-1(facteur de croissance 1 analogue à l'insuline) ;
dans lequel l'hydrogel est un hydrogel choisi dans le groupe consistant en la colle de fibrine, l'acide hyaluronique, la gélatine, l'acide alginique, la cellulose et la pectine ; et
dans lequel le support non dégradable est choisi parmi une fibre d'étoffe non tissée stérilisée, un film PET (polyéthylène téréphtalate), un film PE (polyéthylène), un film PP (polypropylène) ou leurs combinaisons.

2. Procédé selon la revendication 1, dans lequel la colle de fibrine comprend du fibrinogène dans une concentration de 0,5 à 30 mg/mL.

3. Procédé selon la revendication 1, dans lequel la colle de fibrine comprend du fibrinogène dans une concentration de 0,5 à 10 mg/mL.

4. Procédé selon la revendication 1, dans lequel la colle de fibrine comprend de la thrombine dans une concentration de 1 à 50 U.I./mL.

5. Procédé selon la revendication 1, comprenant en outre l'étape (d) d'activation des cellules à l'étape (c) en fournissant au moins un stimulus choisi dans le groupe consistant en un stimulus physique, un stimulus hypoxique, un stimulus mitogène et un stimulus de facteur inflammatoire.

6. Procédé selon la revendication 1, la plaie est une plaie diabétique.

7. Procédé selon la revendication 1, comprenant en outre l'étape (e) de cryoconservation du support-cellules souches mésenchymateuses dérivées de tissu adipeux-hydrogel à l'étape (c) dans un agent de cryoconservation comprenant 1 à 20 % p/v de DMSO (diméthylsulfoxyde) et 1 à 50 % p/v de sérumalbumine humaine, dans lequel le taux de survie des cellules souches mésenchymateuses dérivées de tissu adipeux est de 90 % ou plus après avoir été décongelées.

8. Composition pour la régénération de la peau ou la cicatrisation de plaies, comprenant des cellules souches mésenchymateuses dérivées de tissu adipeux, un hydrogel et un support non dégradable,
dans laquelle l'hydrogel comporte des facteurs de croissance favorisant une croissance cellulaire et une angiogenèse, et une matrice extracellulaire sécrétée par les cellules souches mésenchymateuses lorsque les cellules souches mésenchymateuses, l'hydrogel et le support sont mis en culture dans un milieu d'expansion ;
dans laquelle l'hydrogel est un hydrogel choisi dans le groupe consistant en la colle de fibrine, l'acide hyaluronique, la gélatine, l'acide alginique, la cellulose et la pectine ; et
dans laquelle le support non dégradable est choisi parmi une fibre d'étoffe non tissée stérilisée, un film PET (polyéthylène téréphtalate), un film PE (polyéthylène), un film PP (polypropylène) ou leurs combinaisons.

9. Composition selon la revendication 8, dans laquelle la colle de fibrine comprend du fibrinogène dans une concentration de 0,5 à 30 mg/mL.

10. Composition selon la revendication 8, dans laquelle la colle de fibrine comprend du fibrinogène dans une concentration de 0,5 à 10 mg/mL.

11. Composition selon la revendication 8, dans laquelle la colle de fibrine comprend de la thrombine dans une concentration de 1 à 50 U.I./mL.

12. Composition selon la revendication 8, la plaie est une plaie diabétique.

13. Feuille pour la régénération de la peau ou la cicatrisation de plaies, comprenant l'une de la composition des revendications 8 à 12 en tant que composant actif.
